# EUROPEAN PATENT APPLICATION

(11) **EP 2 343 012 A1**
(43) Date of publication of application: **13.07.2011**
(21) Application number: 09015495.6
(22) Date of filing: 15.12.2009
(51) Int. Cl.: A61B 5/11, G01S 3/02, G08B 23/00

(54) **Security monitor method utilizing a RFID tag and the monitor apparatus for the same**

(71) Applicant: Champtek Incorporated, Hsin-Tien City 231 Taipei (TW)
(72) Inventor: Wang, Cheng-Yi, Hsin Tien City 231 Taipei (TW); Pang, Li-Dar, Hsin Tien City 231 Taipei (TW)
(74) Representative: TER MEER - STEINMEISTER & PARTNER GbR

(57) **Abstract**

A security monitor method and monitor apparatus utilizing a RFID tag. A wearer wears a RFID tag having an acceleration sensor and a magnetic field sensor. The status of the wearer is determined by an acceleration variation and an angle variation of an included angle with the terrestrial magnetism on X axis, Y axis and Z axis detected and calculated by the acceleration sensor and the magnetic field sensor. If the status determined is abnormal, the monitor apparatus transmits wireless warning signals to monitor ends such as a hospital or a home for the aged..

## Description

### BACKGROUND OF THE INVENTION

### Field of the Invention

The invention generally relates to an RFID tag, in particular, to a security monitor method and monitor apparatus utilizing a RFID tag.

### Description of Prior Art

Emergency buttons are widely applied seen in everyday life. There are emergency buttons installed in vehicles and buildings as means for instant alarm and establishing real time communication on site with the competent authorities should emergent incidents occur.

Emergency buttons in a hospital or a home for the aged are installed on the wall or bedside in a ward, a room, or a toilet, such that a patient, a carer, or an old person can press on the emergency buttons in case emergent incident occurs to the patient or the old person to seek for immediate medical assistance from a medical station or a service station. Further, there are additional designs such as an extended cable from an emergency button installed with an extended emergency button on one end so that a patient or an old person can easily reach the extended button when emergent incidents occur. Such designs are especially convenient to a disabled pressing the extended buttons electrically connected to the extended cable to call for help from working or medical staff in a medical station or a service station as emergency incidents occurs.

Though, the above mentioned designs of emergency buttons are provided to offer convenience for patients, carers or old persons to inform medical or service staff, the emergency buttons are located a certain distance away from the floor. In scenarios when patients or old persons fall and unable to stand up, the patients or old persons can not reach the emergency buttons and seek assistance from the medical or service staff.

### SUMMARY OF THE INVENTION

The objective of the prevent invention is to provide a security monitor method and monitor apparatus utilizing a RFID tag. A monitored person wears the monitor apparatus. The sensor or sense device of the monitor apparatus senses the action of the wearer and transmits warning signals with wearer's identification and location information to a monitor end should abnormal actions detected.

In order to realize the above purpose, a RFID tag is used as a monitor apparatus. An acceleration sensor and a magnetic field sensor of the monitor apparatus are used for respectively sensing and calculating and an angle variation of an included angle with the terrestrial magnetism on X axis, Y axis and Z axis as the basis to determine if the wearer is on an abnormal status such as falling. If the wearer is under abnormal status, the monitor apparatus transmits wireless signals to a monitor end such as a hospital or a home for the aged.

The advantage provided by the present invention is the monitor apparatus automatically sensing current status of the wearer in a routine and constantly providing feedbacks to a monitor end. Under the circumstance the wearer is under abnormal status such as falling, the monitor apparatus does not only automatically report to a monitor end by warning signals, also the warning signals further include wearer's identification and wireless positioning location ID to assure the monitor end receive the wearer's identification and location and is allowed to offer immediate assistance to arrive on the location where the wearer is

### BRIEF DESCRIPTION OF DRAWING

The features of the invention believed to be novel are set forth with particularity in the appended claims. The invention itself, however, may be best understood by reference to the following detailed description of the invention, which describes an exemplary embodiment of the invention, taken in conjunction with the accompanying drawings, in which:
Fig. 1 illustrates a schematic diagram of a monitor apparatus according to the present invention;
Fig. 2 illustrates block diagrams of a preferred embodiment according to the present invention; and
Fig. 3 illustrates a monitor flow chart of a embodiment according to the present invention

### DETAILED DESCRIPTION OF THE INVENTION

In cooperation with attached drawings, the technical contents and detailed description of the present invention are described thereinafter according to preferable embodiments.

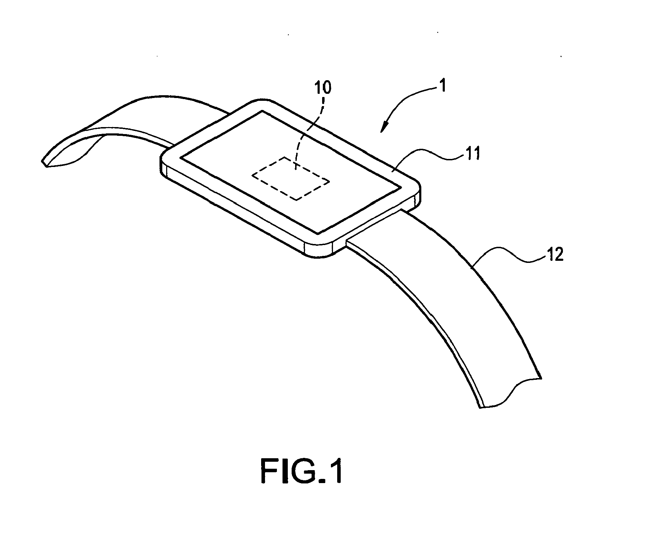

Fig. 1 illustrates a schematic diagram of a monitor apparatus according to the present invention. As shown in the diagram, a monitor apparatus 1 is implemented by a radio frequency identification (RFID) tag 10. The RFID tag 10 is installed in a casing 11. The monitor apparatus 1 comprises the casing 11 and a carrying belt 12 connected to the casing 11 such that a wearer can wear the monitor apparatus 1. With the belt design, a wearer may wear the monitor apparatus 1 on wrist, waist, or neck as the wearer needs. The monitor apparatus 1 may be implemented in forms of a watch, waist belt, and a necklace. It should be noted that the monitor apparatus 1 is used for monitor action status of a patient or an old person. The monitor apparatus 1 uses the carrying belt 12 made mainly by flexible plastic or rubbers but the scope of the invention is not restricted to the listed materials above.

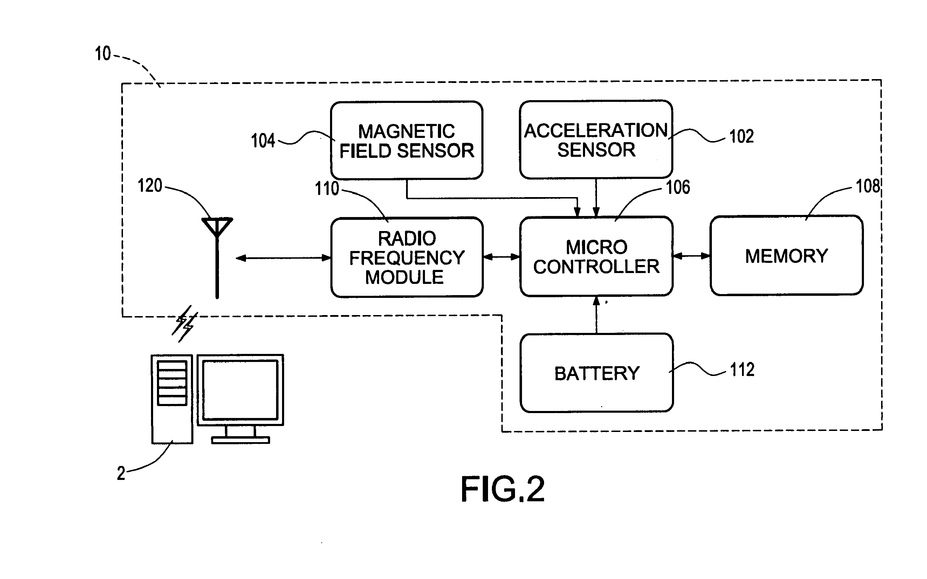

The monitor apparatus 1 senses signal variation on three axes (X axis, Y axis and Z axis) of the wearer caused by wearer's movement via internal sensors installed in the RFID tag 10 (an acceleration sensor 102 and a magnetic field sensor 104 as shown in Fig. 2). Accordingly, when the wearer's status is abnormal (for example the wearer falls and the instant variation of sense signals exceed threshold values), the monitor apparatus transmits a warning signal to a monitor end 2 along with identification ID of the wearer and a wireless positioning location ID of the monitor apparatus 1. Thus, the monitor end 2 is provided with sufficient information to send support persons to the wearer's end immediately to proceed to following confirmation and provide first aid for the emergent incident.

Fig. 2 illustrates block diagrams of a preferred embodiment according to the present invention. As shown in the diagram, the preferred embodiment is implemented by a monitor apparatus 1 having a RFID tag 10 installed inside.

The RFID tag 10, the main component of the monitor apparatus 1 comprises an acceleration sensor 102, a magnetic field sensor 104, a micro controller 106, a memory 108 and a radio frequency module 110. The micro controller 106 is electrically connected to the above components. The radio frequency module 110 is further electrically connected to a radio frequency antenna 120. The RFID tag 10 transmits generated signals via the radio frequency antenna 120 to the monitor end 2. The monitor end 2 receives the signals. In addition to the above components, the RFID tag 10 further comprises a battery 112 as internal power provided to components installed in the RFID tag 10 when the RFID 10 does not transmit and receive signals with the monitor end 2 and thus do not receive external power.

The acceleration sensor 102 of the RFID tag 10 is used for sensing and outputting acceleration signals on X axis, Y axis and Z axis of the wearer. The magnetic field sensor 104 is used for sensing and outputting terrestrial magnetism intensity on X axis, Y axis and Z axis of the wearer to obtain angle signals of an included angle with the terrestrial magnetism. When the wearer wears the monitor apparatus 1, if any of X axis, Y axis and Z axis of the wearer parallel to the spine of the wearer, the angle signals sensed by the magnetic field sensor 104 indicate the spine angle of the wearer. The magnetic field sensor 104 is mainly implemented by a Hall sensor but the he invention is not limited to the implementation.

The above mentioned sensors 102 and 104 constantly sense signals (acceleration signals and angle signals) and output sense signals to the micro controller 106. The micro controller 106 calculates on the sense signal to generate an acceleration variation on three axes of the wearer, and included angle variation between three axes of the wearer and terrestrial magnetism. The micro controller 106 retrieves threshold values saved in advance from the memory 108 and compare the variations with the threshold values. If there's instant dramatic variation sensed on the wearer, the variations exceed the threshold values, the micro controller 106 determines that the wearer is under abnormal status for example the wearer falls.

In addition to the threshold values of the variation, there are wearer identification ID and wireless positioning location ID saved in the memory 108. When emergent incident occurs to the wearer, the identification and location ID signals are transmitted from the micro controller 106 to the radio frequency module 110, encoded by the radio frequency module 110 then transmitted to the monitor end 2 via the radio frequency antenna 120. As such the monitor end 2 for example an hospital, a sanatorium, a residence of the wearer, and a service station or a medical station in the neighboring area of wear's residence is informed of the identification and the incident location of the wearer.

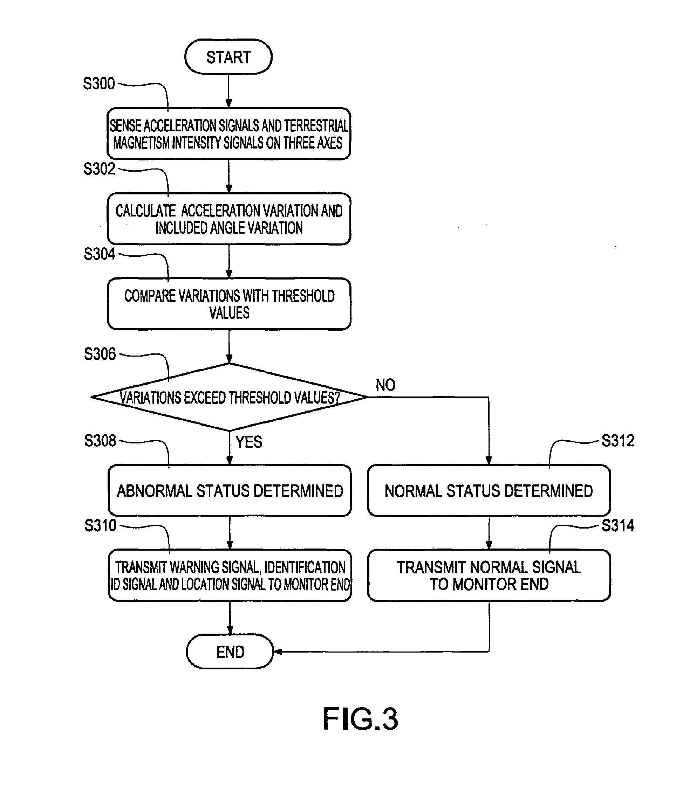

Fig. 3 illustrates a monitor flow chart of an embodiment according to the present invention. Firstly, the monitor apparatus 1 senses acceleration signals on X axis, Y axis, and Z axis via the acceleration sensor 102, and senses terrestrial magnetism intensity signals on X axis, Y axis, and Z axis via the magnetic field sensor 104 for generating an included angle between the X axis, Y axis, and Z axis of the wearer and terrestrial magnetism (step S300). Following that, the acceleration signals and the angle signals are transmitted to the micro controller 106. The micro controller 106 calculates to generate acceleration variation and included angle variation (step S302). The instantaneous applied force is generated by calculating acceleration variation. The spine angle variation of the wearer is generated by calculating included angle variation.

Next, the micro controller 106 compares the variation results with the threshold value saved in the memory 108 (step S304), and determines if the variation results exceed the threshold values (step S306). If the variation results exceed the threshold values, the micro controller 106 determines that the wearer is under abnormal status (step S308), for example the wearer falls and concurrently transmits a warning signal along with signals containing the identification ID, and wireless positioning location ID of the monitor apparatus 1 to the monitor end 2 (step S310). However, if the micro controller 106 determines that the variation results do not exceed the threshold values, the wearer status is determined normal in step S306 (step S312), for example the wearer acts normal or is in sleep. Accordingly, a normal signal is transmitted to the monitor end 2 (step S314). In addition, the above steps are scheduled and operated on a repetitive routine to assure the monitor end 2 receives the wearer status signals continuously. Further, the monitor end 2 is allowed to be informed immediately if emergent incident occurs and sends staff to arrive on the incident location instantly.

## Claims

1. A security monitor method utilizing an RFID tag (10) with an acceleration sensor (102) and a magnetic field sensor (104) as a monitor apparatus (1) for sensing status of a wearer of the monitor apparatus (1), the status of the wearer is determined by the sense results and transmitted to a monitor end (2) via signals, the method comprising:
a) sensing and outputting signals of acceleration on three axes of the wearer by the acceleration sensor (102);
b) sensing and outputting signals of terrestrial magnetism intensity on three axes of the wearer and an included angle with the terrestrial magnetism of the wearer by the magnetic field sensor (104);
c) calculating an acceleration variation according to the acceleration signals;
d) calculating an angle variation according to the angle signals;
e) comparing the acceleration variation and the angle variation with threshold values;
f) determining the status abnormal if the angle variation and the acceleration variation exceed the threshold values following step e;
g) transmitting a warning signal, an ID signal of the wearer and a wireless position location ID signal of the monitor apparatus (1) to the monitor end (2) by the monitor apparatus (1) following step f;
h) determining the status normal if the angle variation and the acceleration variation do not exceed the threshold values following step e; and
i) transmitting a status normal signal to the monitor end (2) by the monitor apparatus (1) following step h.

2. The security monitor method of claim 1, wherein the three axes are X axis, Y axis and Z axis in step a and step b.

3. The security monitor method of claim 2, wherein the acceleration variation is calculated by a micro controller (106) of the monitor apparatus (1) in step c and is used for determining the instantaneous applied force on the wearer.

4. The security monitor method of claim 3, wherein one out of three axes is parallel to the spine of the wearer, and the angle variation is calculated by the micro controller (106) in step d is used for determining a spine angle of the wearer.

5. The security monitor method of claim 1, wherein the status abnormal in the step f refers to that the wearer falls.

6. The security monitor method of claim 5, wherein the status normal in the step h refers to that the wearer acts normal or is in sleep.

7. A security monitor apparatus (1) utilizing a RFID tag (10), the monitored person wearing the security monitor apparatus (1), a status of the wearer being determined by sensing actions of the wearer, and signals being transmitted to a monitor end (2), the monitor apparatus (1) comprising:
a casing (11);
a acceleration sensor (102) installed in the casing (11) and used for sensing and outputting signals of acceleration on three axes of the wearer;
a magnetic field sensor (104) installed in the casing (11) and used for sensing and outputting signals of terrestrial magnetism intensity on three axes of the wearer and an included angle with the terrestrial magnetism of the wearer;
a micro controller (106) installed in the casing (11), electrically coupled to the acceleration sensor (102) and the magnetic field sensor (104), and used for receiving the acceleration signals and the angle signals and calculating a acceleration variation and a angle variation;
a memory (108)installed in the casing (11), electrically coupled to the micro controller (106), and used for saving threshold values of acceleration variation and angle variation;
a radio frequency module (110) installed in the casing (11), electrically coupled to the micro controller (106), and is used for receiving and coding signals generated by the micro controller (106); and
a radio frequency antenna (120) installed in the casing (11), electrically coupled to the frequency module (110), and used for receiving signals and wireless transmitting signals coded by the radio frequency module (110) to the monitor end (2);
wherein, the micro controller (106) compares the acceleration variation and the angle variation with the threshold value, and transmits signals to the monitor end (2) according to the comparing result.

8. The monitor apparatus (1) of claim 7, wherein the monitor apparatus (1) further comprises a battery (112) electrically coupled to the micro controller (106) providing power to the monitor apparatus (1).

9. The monitor apparatus (1) of claim 7, wherein the three axes are X axis, Y axis and Z axis in step a and step b.

10. The monitor apparatus (1) of claim 7, wherein one out of three axes is parallel to the spine of the wearer, and the angle variation is calculated by the micro controller (106) in step d is used for determining a spine angle of the wearer.

11. The monitor apparatus (1) of claim 10, wherein the magnetic field sensor (104) is a Hall sensor based on Hall effect.

12. The monitor apparatus (1) of claim 10, wherein the wearer carries a carrying belt (12) connected to the casing (11) to assure the monitor apparatus (1) is positioned on the wearer.

13. The monitor apparatus (1) of claim 12 wherein the carrying belt (12) is made of flexible plastic or rubber to allow the wearer to wear the monitor apparatus (1) on wrist, waist or neck.

14. The monitor apparatus (1) of claim 7, wherein the monitor end (2) refers to a hospital, a sanatorium, a residence of the wearer, and a service station or a medical station in the neighboring area of wear's residence.
